# EUROPEAN PATENT SPECIFICATION

(11) **EP 4 424 264 B1**
(45) Date of publication and mention of the grant of the patent: **24.12.2025**
(21) Application number: 22886370.0
(22) Date of filing: 27.05.2022
(51) Int. Cl.: A61B 34/37, A61F 9/007, B25J 3/00, A61B 1/00

(54) **FOOT CONTROLLER AND SURGERY ASSISTANCE DEVICE**
FUSSSTEUERUNG UND CHIRURGIEASSISTENZVORRICHTUNG
DISPOSITIF DE COMMANDE À PIED ET DISPOSITIF D'ASSISTANCE CHIRURGICALE

(30) Priority: 27.10.2021 JP 2021175185
(43) Date of publication of application: 04.09.2024
(73) Proprietor: RIVERFIELD Inc., Tokyo 107-0052 (JP)
(72) Inventor: FUKUDA, Tatsuhito, Tokyo 160-0017 (JP); SONODA, Koh-hei, Fukuoka-shi, Fukuoka 819-0395 (JP)
(74) Representative: Adares PartGmbB
(86) International application number: PCT/JP2022/021762
(87) International publication number: WO 2023/074033

(56) References cited:
- EP-B2- 0 653 922
- WO-A1-2018/100828
- DE-A1- 102013 015 109
- JP-A- 2002 153 487
- JP-A- 2005 204 999
- JP-A- 2019 134 914
- JP-A- 2021 049 633
- US-A1- 2019 239 971
- US-A1- 2020 008 896

## Description

### Technical Field

The present invention relates to a surgery assistance device having to a foot controller, such as a foot controller used for operation and control of medical surgical tools during surgery.

### Background Art

Pedals and foot controllers for operators, such as doctors and assistants, using surgical tools to perform part of operation and control of the surgical tools with their foot in various surgical operations have been devised. For example, Patent Literature 1 teaches a foot controller having a switch that enables operations in a first mode by being pressed down by toes and operations in a second mode by being pressed horizontally by a foot side.

Further, Patent Literature 2 describes a food control panel for a medical device having a several operation parts in the form of knobs, a panel element or rocker switch, and a joystick. Document EP 0 653 922 B2 discloses a surgery assistant device comprising an endoscope; a holding member for holding the endoscope; an arm part for adjusting a position of the holding member; a display configured to display a visual field image taken by the endoscope; a foot controller; and a controller configured to change the visual field image in accordance with an operation on the foot controller, wherein the foot controller comprises a placement part on which a foot is to be placed; a first operating part capable of being operated back and forth and left and right by the foot placed at a first position of the placement part; and a second operating part capable of being operated back and forth by the foot placed at a second position of the placement part only.

### Related Art List

### Patent Literature

Patent Literature 1: JP 2012-183382 A
Patent Literature 2: DE 10 2013 015109 A1

### Summary of Invention

### Technical Problem

According to the switch of the aforementioned foot controller according to Patent Literature 1, however, up to two operation modes can be selected with one switch. Thus, more switches will be needed for enabling selection of more operation modes, which makes the foot controller have a more complicated structure and a larger size. Furthermore, in operation of the aforementioned switch, the position of the foot that presses the switch to switch the mode needs to be changed, which may lower the user-friendliness. There is therefore room for further improvement in terms of reducing the operational burden and the operational errors of the operator.

The present invention has been made in view of the aforementioned circumstances, and an exemplary object thereof is to provide a novel foot controller that is both space-saving and user-friendly.

### Solution to problem

To solve the aforementioned problems, a surgery assistance device according to claim 1 is provided. The foot controller is a foot controller to be used for operation of a device and includes: a placement part on which a foot is to be placed; a first operating part capable of being operated back and forth and left and right by the foot placed at a first position of the placement part; and a second operating part capable of being operated back and forth and left and right by the foot placed at a second position of the placement part. On the placement part, the first operating part and the second operating part are arranged along a front-back direction, and a predetermined operation of the device is selected in accordance with a direction of an operation on the first operating part or the second operating part.

According to this aspect, the foot controller can have a reduced width. Furthermore, because a number of operations of a device can be selected with two operating parts, the foot controller has improved user-friendliness and a reduced size. Note that the back and forth and left and right operations of each operating part need not necessarily be set in such a manner that each of all the operations of the operating part is associated with selection of a predetermined operation of the device, but some operations may be invalid, that is, some operations the operating part may be associated with none of the operations of the device.

The first operating part is located at a recess on the placement part, and the second operating part is located at a position that does not interfere with the foot placed at the first position. As a result, it is unlikely that the second operating part is erroneously touched while the first operating part is being operated with a foot.

The second operating part may be located at a higher position than the first operating part, and the first operating part may be located at a position that does not interfere with the foot placed at the second position. As a result, it is unlikely that the first operating part is erroneously touched while the second operating part is being operated with a foot.

A third operating part on a side of the placement part may further be included. The third operating part may be capable of being operated by a horizontal movement of the foot placed at the first position and also capable of being operated by a horizontal movement of the foot placed at the second position. Thus, the third operating part can be operated by a horizontal movement of a foot both in the case where the foot is placed at the first position and in the case where the foot is placed at the second position.

The third operating part may be a pair of switches at respective sides of the placement, and the first operating part and the second operating part may be located in a region between the pair of switches. Thus, a switch can be operated by a horizontal movement of the foot in either direction, which enables an operation without a sense of discomfort as compared with a case where a switch is provided only on one side of the placement part even if there is a difference in easiness of horizontal movement of a foot or a difference in easiness between a rightward movement and a leftward movement of a foot depending on the operator's dominant foot. This allows the foot controller to be operated by both of left and right feet.

The first operating part and the second operating part may be joysticks. This allows relatively easy back and forth and left and right operations with a sole.

The surgery assistance device includes: an endoscope; a holding member for holding the endoscope; an arm part for adjusting a position of the holding member; a display configured to display a visual field image taken by the endoscope; the aforementioned foot controller; and a controller configured to change the visual field image in accordance with an operation on the foot controller.

According to this aspect, because the visual field image can be changed in accordance with the operation on the foot controller, interruption of manual work of the operator to change the visual field image can be avoided.

The controller may include a drive controller configured to control movement of the endoscope through the arm part so that a visual field of the endoscope moves in accordance with a back/forth/left/right operation of the first operating part. Thus, the visual field can be moved without regard to the movement of the endoscope.

The controller includes: a drive controller configured to control movement of the endoscope through the arm part so that the visual field of the endoscope zooms in and out in accordance with the back and forth operation of the second operating part; and an image processor configured to perform image processing so that the visual field image turns in accordance with a left/right operation of the second operating part. Thus, operation of the second operating part is only needed to cause the visual field to zoom in and out without regard to the movement of the endoscope, and the visual field image can be turned by image processing without moving the endoscope.

A non-inventive embodiment is another surgery assistance device. The surgery assistance device includes: an endoscope; a holding member for holding the endoscope; an arm part for adjusting a position of the holding member; a retracting mechanism for retracting the endoscope from an eyeball; and the aforementioned foot controller. When the third operating part on the foot controller is operated, the retracting mechanism retracts the endoscope from the eyeball.

According to this aspect, in an emergency, the operator only needs to move his/her foot horizontally to retract the endoscope from an eyeball, for example.

Note that any combination of the components described above, and any expression in the present invention converted to that for a method, a device, a system, and the like also remain as aspects of the present invention.

### Advantageous Effects of Invention

According to the present invention, a foot controller that is both space-saving and user-friendly can be achieved.

### Brief Description of Drawings

FIG. 1A is a top view schematically illustrating a configuration of a surgical system, and FIG. 1B is a side view schematically illustrating the configuration of the surgical system.
FIG. 2 is a diagram schematically illustrating a cross section structure of an eyeball.
FIG. 3 is a schematic diagram illustrating a surgery assistance device according to the embodiment.
FIG. 4 is a side view of an outline structure in a state in which an endoscope is inserted in an eyeball by a retracting mechanism according to the embodiment.
FIG. 5 is a side view of an outline structure in a state in which the endoscope is retracted from the eyeball by the retracting mechanism according to the embodiment.
FIG. 6 is a perspective view of a foot controller according to the embodiment.
FIG. 7A is a top view of the foot controller according to the embodiment, and FIG. 7B is a side view of the foot controller according to the embodiment.
FIG. 8A is a perspective view of the foot controller in a state in which a foot is placed at a first position of a placement part, and FIG. 8B is a side view of the foot controller in the state in which the foot is placed at the first position of the placement part.
FIG. 9A is a perspective view of the foot controller in a state in which a foot is placed at a second position of the placement part, and FIG. 9B is a side view of the foot controller in a state in which the foot is placed at the second position of the placement part.
FIG. 10 is a top view of the foot controller in a state in which a foot is placed on the placement part.
FIG. 11 is a block diagram of a surgery assistance device according to the embodiment.
FIG. 12A is a schematic diagram illustrating an operation of a first joystick, and FIG. 12B is a schematic diagram for explaining a change of visual field image corresponding to the operation of the first joystick illustrated in FIG. 12A.
FIG. 13A is a schematic diagram illustrating an operation of a second joystick, and FIG. 13B is a schematic diagram for explaining a change of visual field image corresponding to the operation of the second joystick illustrated in FIG. 13A.

### Description of Embodiments

The present invention will now be described on the basis of an embodiment with reference to the drawings. Components, members, and processes that are the same as or equivalent to each other illustrated in the drawings are represented by the same reference numerals, and redundant explanation will not be repeated where appropriate. The embodiment is not to limit the invention, but is an example, and any feature or any combination of features described in the embodiment is not necessarily essential to the invention.

### [Surgical system]

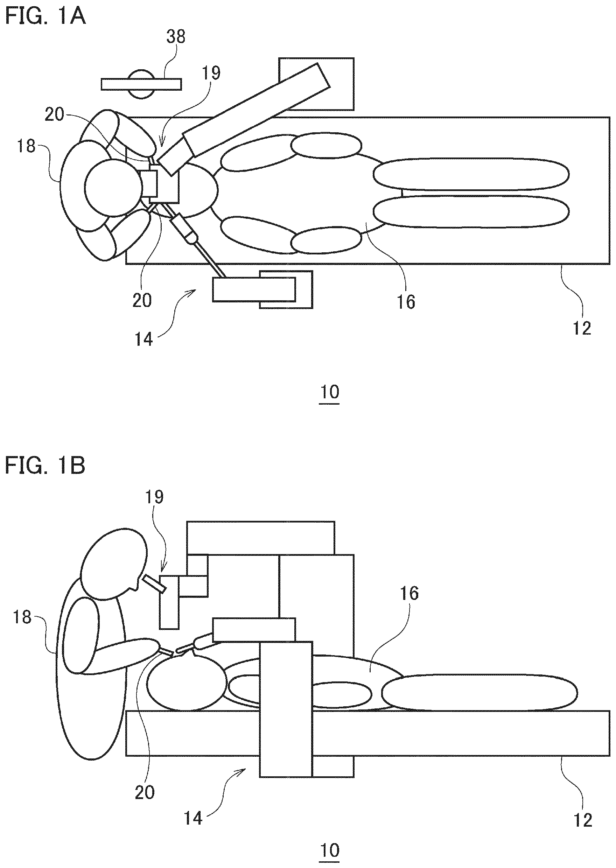

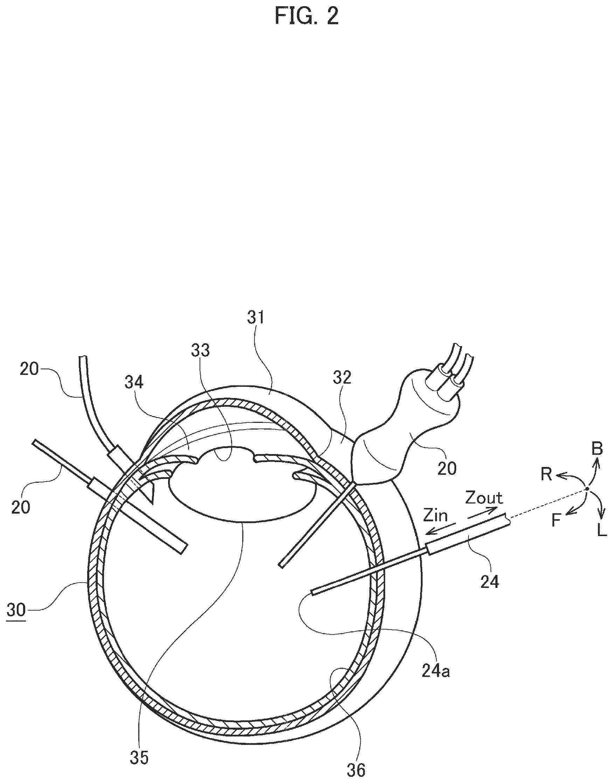

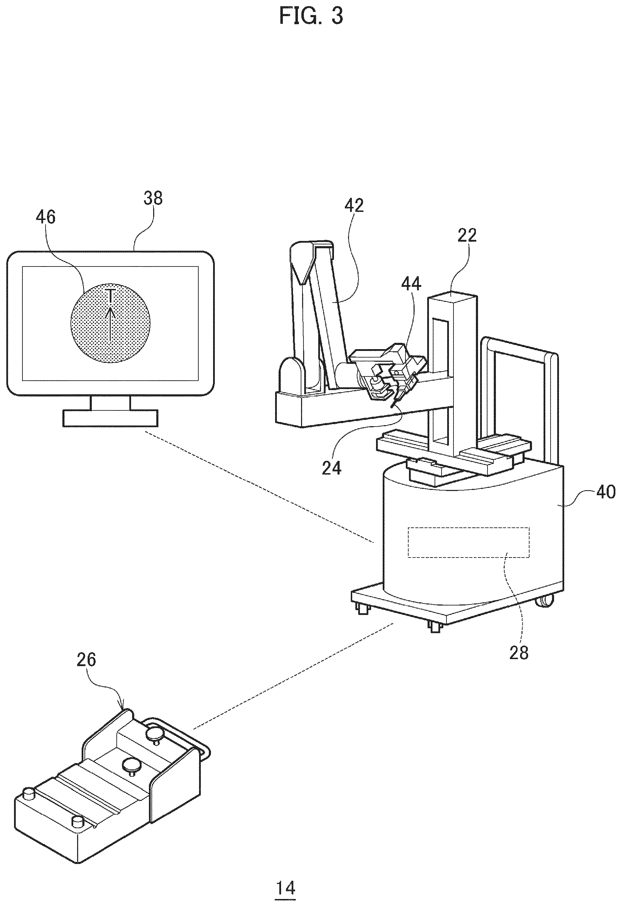

An embodiment will be described below with reference to an example of a surgical system to be used in intraocular surgery. FIG. 1A is a top view schematically illustrating a configuration of the surgical system, and FIG. 1B is a side view schematically illustrating the configuration of the surgical system. FIG. 2 is a diagram schematically illustrating a cross section structure of an eyeball. FIG. 3 is a schematic diagram illustrating a surgery assistance device according to the embodiment.

A surgical system 10 illustrated in FIGS. 1A and 1B includes an operating table 12, and a surgery assistance device 14. A patient 16, who is a subject of an operation, is laid on his/her back on the operating table 12. A doctor 18, who is a surgeon, takes his/her position at the head of the patient 16, and performs an operation inside an eyeball 30 (see FIG. 2) of the patient 16 by using an operating microscope 19 and various treatment tools 20. Examples of the treatment tools 20 include a vitreous cutter, forceps, and an injector for irrigating solution or the like.

The surface of the eyeball 30 illustrated in FIG. 2 is covered with a cornea 31 and a conjunctiva 32, an iris 34 with a pupil 33 is present behind the cornea 31, and a crystalline lens 35 is present behind the iris 34. In addition, a retina 36 is present over an eyeground inside the eyeball 30. The doctor 18 performs an operation inside the eyeball 30 by inserting a treatment tool 20 into the conjunctiva 32, for example.

### [Surgery assistance device]

The operation on the eyeball 30 performed by the doctor 18 is assisted by the surgery assistance device 14. The surgery assistance device 14 illustrated in FIG. 3 includes an endoscope holder 22, an endoscope 24, a foot controller 26, a controller 28, and a monitor 38, which is a display. The endoscope holder 22 includes a base part 40 and an arm part 42. The base part 40 is placed on a floor of an operating room or the like, and the arm part 42 is attached to the base part 40. The arm part 42 is turnably supported by the base part 40.

The arm part 42 is a mechanism including one or more joint portions and turnable portions and being capable of moving a holding member 44 at an arm distal end to an intended position with an intended posture. This enables control of the movement of the endoscope 24 held by the holding member 44 through the arm part 42. In other words, the endoscope 24 can be freely moved to any intended position by moving the arm part 42 with the endoscope 24 fixed to the holding member 44.

The endoscope 24 inserted in the eyeball 30 of the patient 16 can be held by the holding member 44, which eliminates the need for the doctor 18 to hold the endoscope 24 with a hand. The doctor 18 can therefore use both hands to perform the operation on the eyeball 30.

The endoscope 24 of the surgery assistance device 14 is inserted into the eyeball 30 in a state in which the endoscope 24 is fixed to the holding member 44 (see FIG. 2). The state inside the eyeball 30 is imaged by the inserted endoscope 24. Image signals obtained by an image sensor of the endoscope 24 are converted through analog-to-digital conversion into digital image signals (image data) representing luminance of a predetermined number of gradations in units of pixels. An image based on the image data from the endoscope 24 is displayed on the monitor 38.

The foot controller 26 is an operation device used for operation of the movement of the endoscope 24 through the arm part 42, and zooming in, zooming out, and rotation of an image, which was taken by the endoscope 24, displayed on the monitor 38, and the like. Some operating parts of the foot controller 26 may be a joystick, a trackball, and a D-pad.

The controller 28 performs various processes necessary for implementing the surgery assistance device 14 according to the embodiment, such as control on the operation of the arm part 42, processes of generating various images to be displayed on the monitor 38, and processes of controlling the display on the monitor 38.

The controller 28 includes a microcomputer including a central processing unit (CPU), a read only memory (ROM), a random access memory (RAM), and the like, for example. The controller 28 is constituted by one or more microcomputers. The controller 28 is included in the base part 40 of the endoscope holder 22, for example. Alternatively, the controller 28 may be included in another external device.

The monitor 38 displays a visual field image 46 of the endoscope 24 on the basis of the display control performed by the controller 28. A direction T toward the top is a direction opposite the gravitational direction, and the upward direction of the visual field image 46 on the monitor 38 is adjusted to correspond to the direction T toward the top at initial settings.

### [Retracting mechanism]

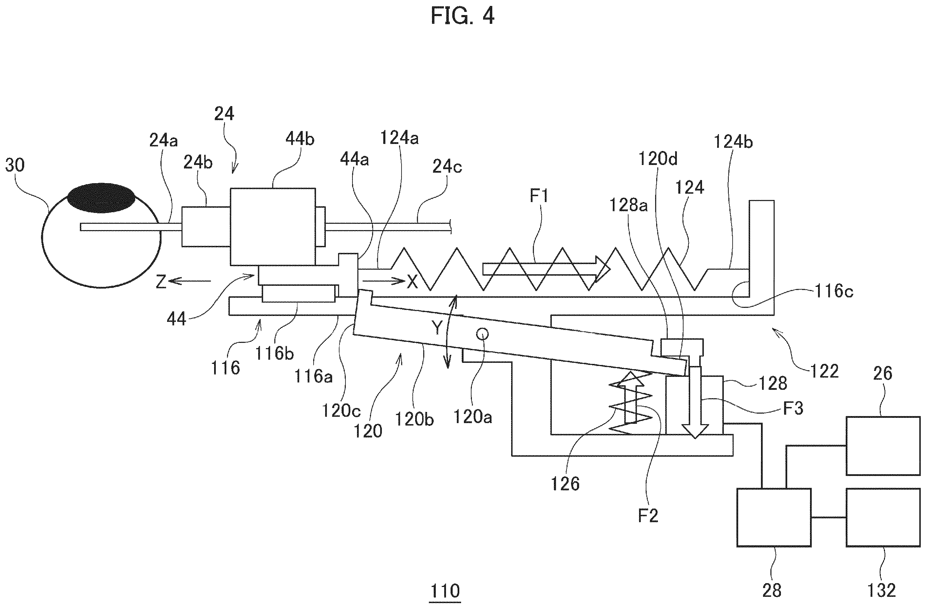

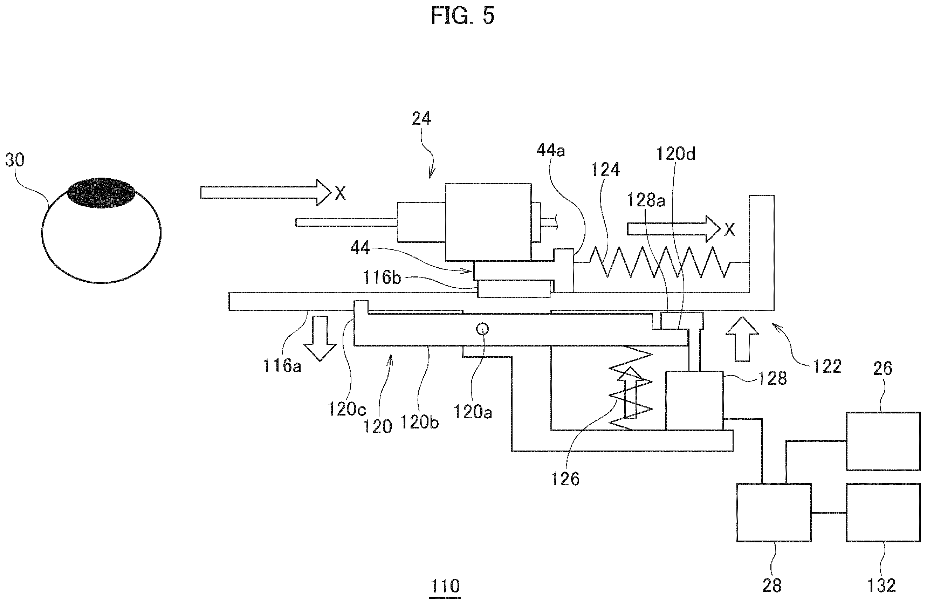

Next, a retracting mechanism for retracting the endoscope inserted in the eyeball in an emergency will be described. FIG. 4 is a side view of an outline structure in a state in which an endoscope is inserted in an eyeball by a retracting mechanism according to the embodiment. FIG. 5 is a side view of an outline structure in a state in which the endoscope is retracted from the eyeball by the retracting mechanism according to the embodiment. Note that the retracting mechanism according to the embodiment is provided between the endoscope 24 and the arm part 42.

The retracting mechanism 110 illustrated in FIG. 4 includes the holding member 44 for holding the endoscope 24, which is a surgical tool, a guiding member 116 for guiding the holding member 44 to linearly advance and retract, a locking member 120 for locking the position of the holding member 44 in a state in which the endoscope 24 is inserted in an eyeball 30, which is a surgical site, and a retracting unit 122 for releasing the locking state of the locking member 120 and retracting the holding member 44 in accordance with a signal from outside.

The endoscope 24 includes a fiber-like insertion portion 24a having a diameter of about 0.5 to 1.2 mm to be inserted into an eyeball 30, a grip 24b, and a cable 24c. The grip 24b is held by an adapter 44b of the holding member 44. The guiding member 116 includes a linear guide 116a, and a stage 116b configured to slide along the linear guide 116a.

The retracting unit 122 includes a tension spring 124 that is a first force generating member for generating a first force F1 in a direction X in which the holding member 44 is to be retracted in a state in which the holding member 44 is locked, a compression spring 126 that is a second force generating member for generating a second force F2 for releasing the locking state of the locking member 120, and a pull-type solenoid 128 that is a third force generating member for generating a third force F3 for maintaining, against the second force F2, the locking state of the locking member 120.

A first end 124a of the tension spring 124 is fixed to an end 44a in the retracting direction of the holding member 44. A second end 124b of the tension spring 124 is fixed to an end 116c of the linear guide 116a. The locking member 120 is a rod-like turnable member 120b configured to turn about a fulcrum 120a in a Y direction. The turnable member 120b has a first end 120c that comes in contact with the end 44a of the holding member 44 and a second end 120d on which the third force F3 acts in the locking state.

The pull-type solenoid 128 pulls the second end 120d of the turnable member 120b while power is on. Specifically, the pull-type solenoid 128 has a hook-like catching portion 128a at an end of a plunger thereof. The catching portion 128a fixes the second end 120d of the turnable member 120b in a state in which the second end 120d is pulled downward.

The compression spring 126 of the retracting unit 122 according to the embodiment has a spring constant and a length set so that the locking state of the locking member 120 is released while power supply to the pull-type solenoid 128 is off. In addition, the retracting unit 122 further includes a controller 28 for controlling power supply to the pull-type solenoid 128, a sensor 132 for detecting the movement of the eyeball 30 or the movement of the head, and the foot controller 26.

Next, an operation of immediately retracting the endoscope 24 from the eyeball 30 in an X direction will be explained with reference to FIG. 5. As illustrated in FIG. 5, when the pull-type solenoid 128 is powered off, the third force F3 by which the catching portion 128a of the pull-type solenoid 128 has pulled the second end 120d of the turnable member 120b downward is eliminated. As a result, the second end 120d is pushed upward and the first end 120c, which is located on a side opposite the second end 120d with respect to the fulcrum 120a, is pushed downward by the restoring force of the compression spring 126, and the locking of the holding member 44 by the locking member 120 is thus released.

As a result, the restoring force of the tension spring 124 with the first end 124a fixed to the end 44a of the holding member 44 moves the holding member 44 in the X direction, and the endoscope 24 is retracted from the eyeball 30 in the X direction.

As described above, the retracting mechanism 110 according to the embodiment allows the endoscope 24 linearly inserted in the eyeball 30, which is the surgical site, along the linear guide 116a to be retracted in parallel (in the X direction) with the inserting direction Z of the endoscope 24. As a result, the load applied to the eyeball 30 while the endoscope 24 is retracted from the eyeball 30 is reduced.

In addition, the retracting mechanism 110 achieves holding and retraction of the endoscope 24 by adjusting the forces generated by the tension spring 124, the compression spring 126, and the pull-type solenoid 128 by a simple structure.

In addition, in the retracting mechanism 110, because the pull-type solenoid 128 that requires power supply and a relatively large space can be placed at a position away from the holding member 44, the flexibility of layout of respective components is increased without making the structure complicated.

Furthermore, in such an emergency in which power supply to the pull-type solenoid 128 is cut off owing to a power failure or a device failure, for example, the retracting mechanism 110 is capable of quickly retracting the endoscope 24 from the eyeball 30 without requiring special control. In addition, the retracting mechanism 110 is capable of generating the second force F2 by the restoring force of the compression spring 126 without using power, and similarly capable of generating the first force F1 by the restoring force of the tension spring 124 without using power.

Furthermore, in an emergency, the retracting mechanism 110 is capable of quickly retracting the endoscope 24 inserted in such a soft and delicate surgical site as the eyeball 30 while reducing the load applied to the eyeball 30.

In addition, the controller 28 of the retracting unit 122 according to the embodiment is configured to release the locking state of the locking member 120 by controlling current to turn off power supply to the pull-type solenoid 128 in accordance with a signal in response to a movement of the eyeball or the like detected by the sensor 132. This allows the endoscope 24 held in a state inserted in the eyeball 30 to be quickly retracted before a large load is applied to the eyeball 30 by the movement of the position of the eyeball 30.

While the retracting unit 122 according to the embodiment is a combination of the compression spring 126, which is the second force generating member, and the pull-type solenoid 128, which is the third force generating member, a retracting unit may be configured as a combination of a tension spring, as a second force generating member, for pulling the turnable member 120b upward and a push-type solenoid, as a third force generating member, for pushing the second end 120d of the turnable member 120b downward when power is supplied to the push-type solenoid.

### [Foot controller]

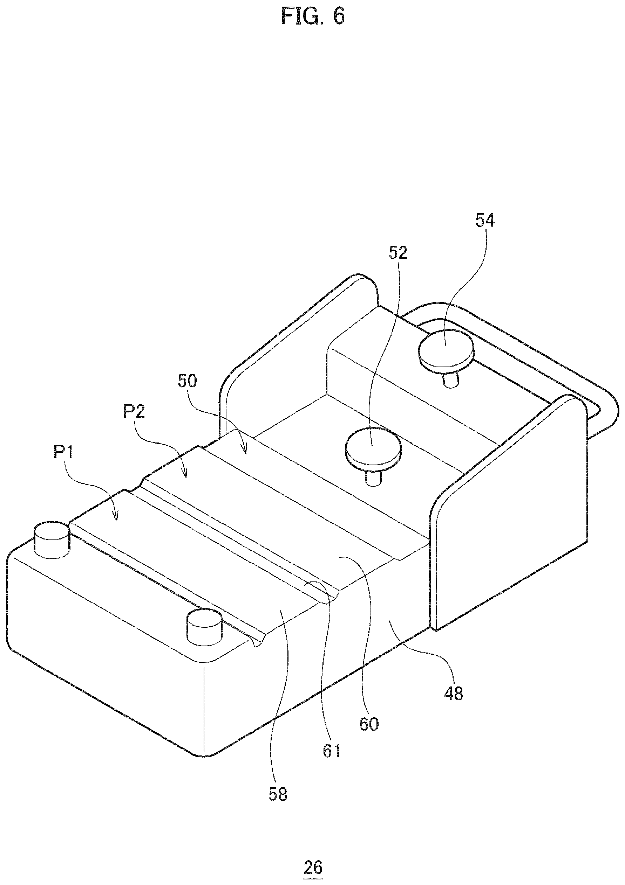

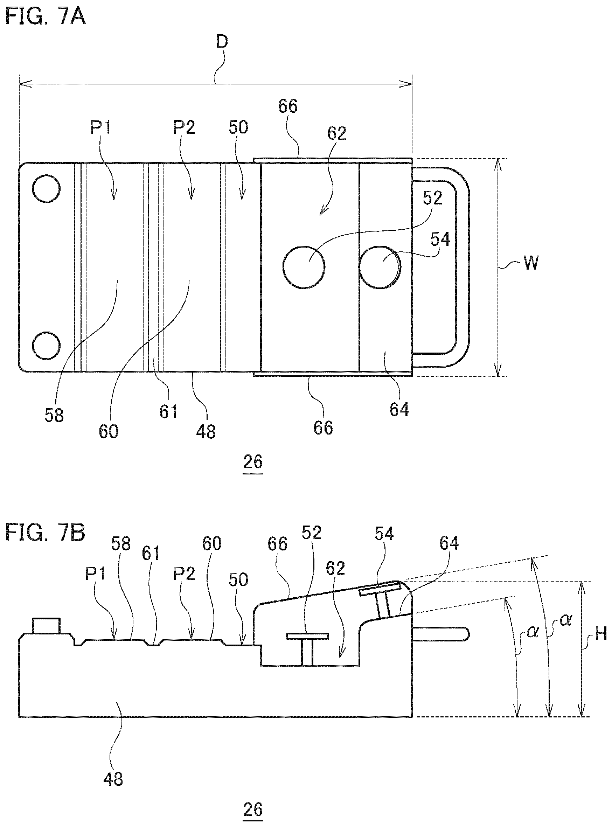

Next, a foot controller according to the embodiment will be described in detail. FIG. 6 is a perspective view of a foot controller according to the embodiment. FIG. 7A is a top view of the foot controller according to the embodiment, and FIG. 7B is a side view of the foot controller according to the embodiment. Note that illustration of a kick switch (to be described later) on a right side face of the foot controller is omitted in FIG. 7B.

The foot controller 26 includes a box-shaped housing 48, and is used for operating the endoscope holder 22. Specifically, the foot controller 26 includes, on the top face of the housing 48, a placement part 50 on which a foot is to be placed, a first joystick 52, which is a first operating part, that can be operated back and forth and left and right by a foot placed at a first position P1 of the placement part 50, and a second joystick 54, which is a second operating part, that can be operated back and forth and left and right by a foot placed at a second position P2 of the placement part 50. The joysticks are of the type that can be relatively easily operated back and forth and left and right by a sole.

The first joystick 52 and the second joystick 54 are arranged along the front-back direction on the placement part 50, and a predetermined operation of the endoscope holder 22 is selected in accordance with the direction of the operation on the first joystick 52 or the second joystick 54. While each joystick according to the embodiment is capable of being operated in at least four directions, the joysticks may be operating parts capable of being operated in more directions.

The length D in the front-back direction of the placement part 50 is preferably larger than the foot length of a typical adult man and smaller than 400 mm. This minimizes the length D in the front-back direction of the foot controller 26. In addition, because the two joysticks are arranged along the front-back direction, the width W of the foot controller 26 can be minimized. The width W is preferably larger than the foot width of a typical adult man and smaller than 250 mm. The height H is about 100 to 150 mm at a highest position of the foot controller 26.

Because a number of operations of the endoscope holder 22 can be selected with two joysticks, the user-friendliness of the foot controller 26 according to the embodiment can be improved and the size of the entire foot controller 26 can be reduced. Note that the back and forth and left and right operations of each joystick need not necessarily be set in such a manner that each of all the operations of the joystick is associated with selection of a predetermined operation of the endoscope holder 22, but some operations may be invalid, that is, some operations the joystick may be associated with none of the operations of the endoscope holder 22.

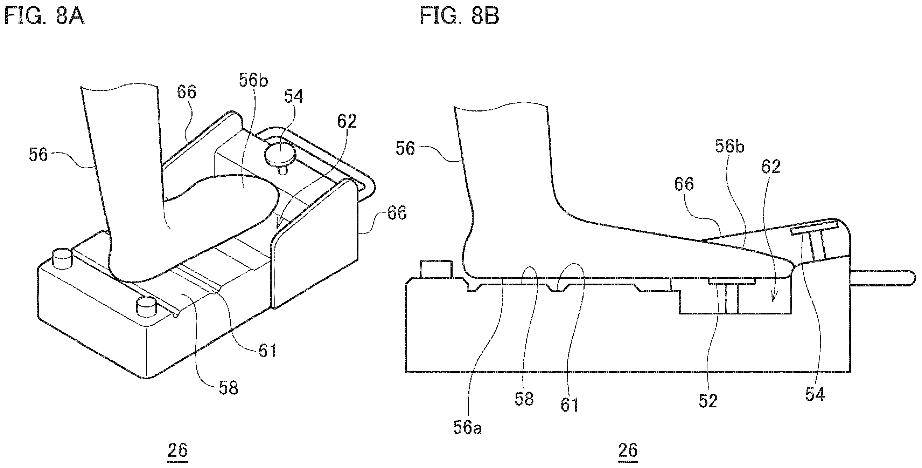

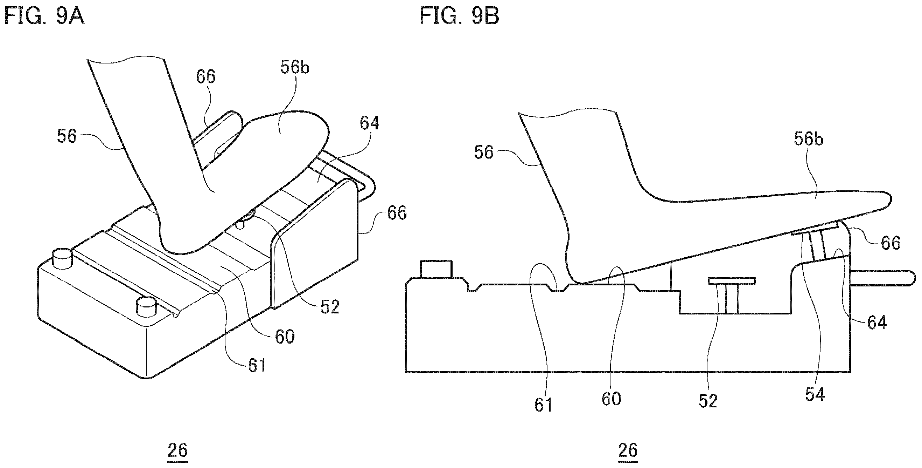

FIG. 8A is a perspective view of the foot controller in a state in which a foot is placed at the first position of the placement part, and FIG. 8B is a side view of the foot controller in the state in which the foot is placed at the first position of the placement part. FIG. 9A is a perspective view of the foot controller in a state in which a foot is placed at the second position of the placement part, and FIG. 9B is a side view of the foot controller in a state in which the foot is placed at the second position of the placement part. Note that illustration of the kick switch (to be described later) on the right side face of the foot controller is omitted in FIGS. 8B and 9B.

The placement part 50 includes a first footrest 58 on which the heel 56a of a foot 56 placed at the first position P1 is placed, a second footrest 60 on which the heel 56a of a foot 56 placed at the second position P2 is placed, and a recess 62 at which the second joystick 54 is located. A groove 61 is formed between the first footrest 58 and the second footrest 60 so as to make it easier to recognize the position at which a foot is to be placed.

As illustrated in FIGS. 8A and 8B, the foot 56 placed at the first position P1 can operate the first joystick 52 with the bottom of the toes 56b. In this state, the second joystick 54 is located at a position that does not interfere with the foot 56 placed at the first position P1. As a result, it is unlikely that the second joystick 54 is erroneously touched while the first joystick 52 is being operated with the foot 56.

According to the foot controller 26 according to the embodiment, the second joystick 54 is located at a position higher than the first joystick 52. In addition, a region of the placement part 50 in which the second joystick 54 is located has a tilted surface 64 with respect to an installation surface on which the foot controller 26 is installed, and the angle between the installation surface and the tilted surface 64 is α° (see FIG. 7B). Note that α° is an angle between 10° and 15°. In addition, a main surface of the placement part 50 and the installation surface are substantially parallel to each other.

As a result, as illustrated in FIGS. 9A and 9B, the foot 56 placed at the second position P2 has a toe-up posture and thus can operate the second joystick 54 with the bottom of the toes 56b. In this state, the first joystick 52 is located at the bottom of the recess 62 so that the first joystick 52 does not interfere with the foot 56 placed at the second position P2. As a result, it is unlikely that the first joystick 52 is erroneously touched while the second joystick 54 is being operated with the foot 56.

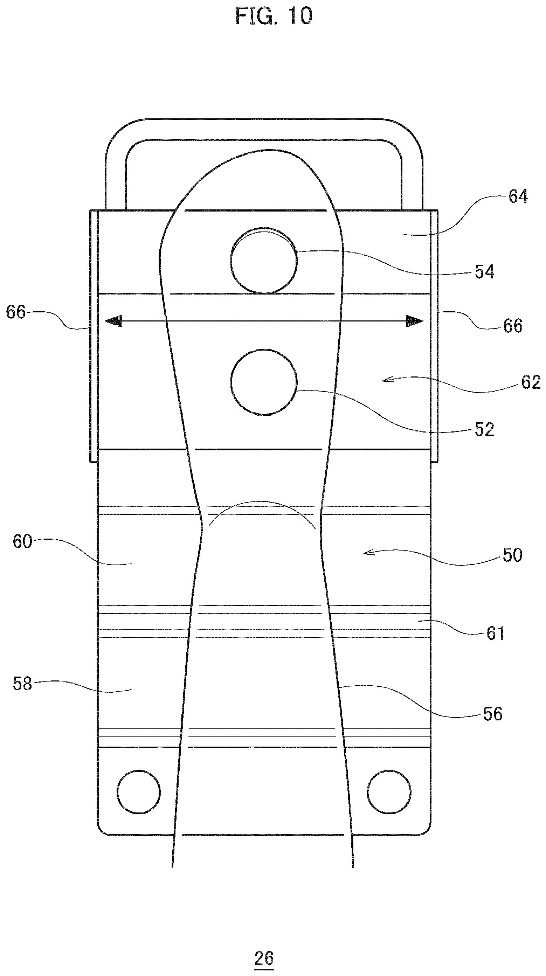

FIG. 10 is a top view of the foot controller in a state in which a foot is placed on the placement part. The foot controller 26 further includes a kick switch 66, which is a third operating part, that is provided on a side of the placement part 50. As illustrated in FIG. 10, the kick switch 66 is capable of being operated by a horizontal movement (in the direction of an arrow) of the foot 56 placed at the first position P1 and is also capable of being operated by a horizontal movement of the foot 56 placed at the second position P2. Thus, the kick switch 66 can be operated by a horizontal movement of the foot 56 both in the case where the foot 56 is placed at the first position P1 and in the case where the foot 56 is placed at the second position P2.

In the embodiment, the kick switch 66 is provided in a form of a pair of kick switches 66 at respective sides of the placement part 50, and the first joystick 52 and the second joystick 54 are arranged in a region between the pair of kick switches 66. Thus, the kick switch 66 can be operated by a horizontal movement of the foot 56 in either direction, which enables quick operation without a sense of discomfort as compared with a case where a kick switch is provided only on one side of the placement part 50 even if there is a difference in easiness of horizontal movement of a foot or a difference in easiness between a rightward movement and a leftward movement of a foot depending on the operator's dominant foot. This allows the foot controller 26 to be operated by both of left and right feet.

### [Operation of endoscope holder by foot controller]

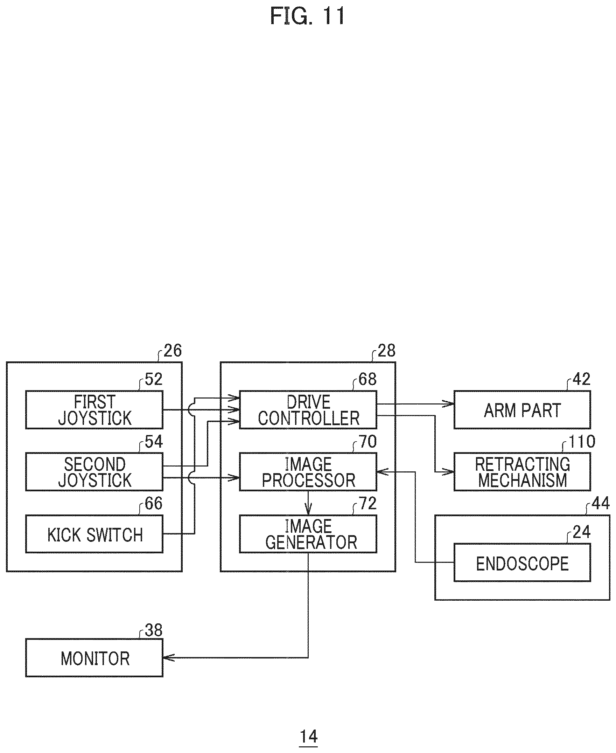

FIG. 11 is a block diagram of the surgery assistance device 14 according to the embodiment. As described above, the surgery assistance device 14 according to the embodiment includes the endoscope 24, the holding member 44 for holding the endoscope 24, the arm part 42 for adjusting the position of the holding member 44, the retracting mechanism 110, the monitor 38 for displaying a visual field image taken by the endoscope 24, the foot controller 26, and the controller 28 that changes the visual field image 46 in accordance with operations performed on the foot controller 26.

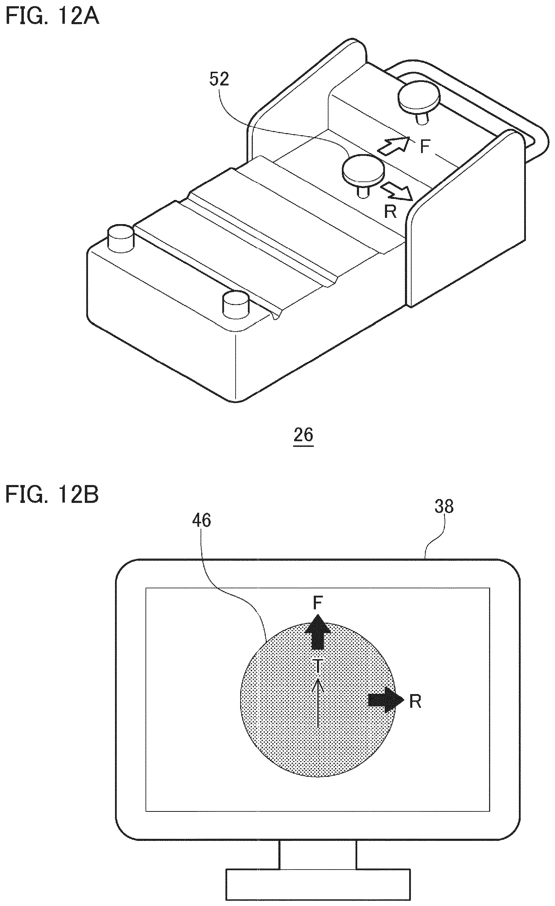

FIG. 12A is a schematic diagram illustrating an operation of a first joystick, and FIG. 12B is a schematic diagram for explaining a change of a visual field image corresponding to the operation of the first joystick illustrated in FIG. 12A. In order to move the visual field on the visual field image 46 back and forth and left and right, a possible way is to change the orientation of the tip of the insertion portion 24a of the endoscope 24 back and forth and left and right.

Thus, a drive controller 68 included in the controller 28 controls the movement of the endoscope 24 through the arm part 42 so that the visual field of the endoscope 24 in accordance with back and forth and left and right operations performed on the first joystick 52. For example, as illustrated in FIG. 2, the visual field of the visual field image 46 can be moved by tilting the endoscope 24 back and forth and left and right (FBLR) around a position at which the endoscope 24 is inserted into the eyeball 30. Thus, because the visual field image 46 can be changed in accordance with the operation of the first joystick 52 on the foot controller 26, interruption of manual work of the doctor 18, who is the operator, to change the visual field image 46 can be avoided.

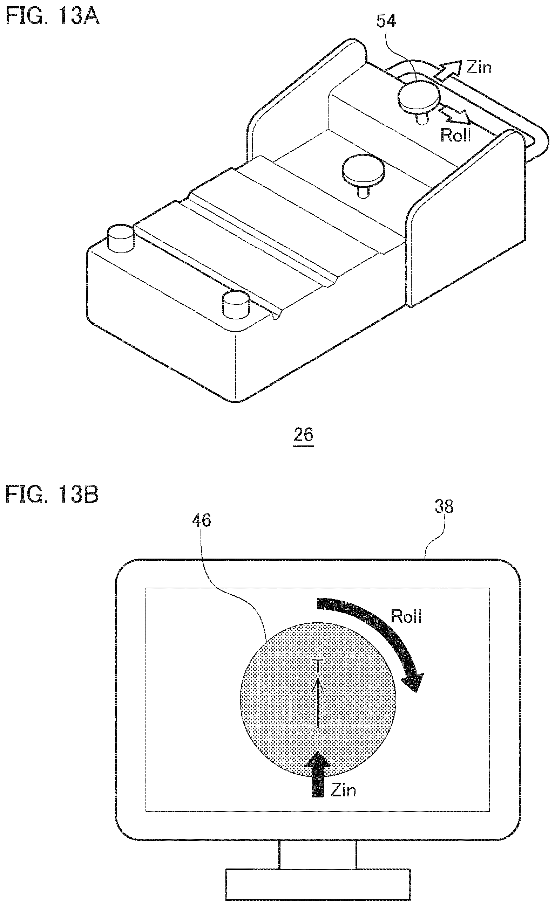

FIG. 13A is a schematic diagram illustrating an operation of a second joystick, and FIG. 13B is a schematic diagram for explaining a change of visual field image corresponding to the operation of the second joystick illustrated in FIG. 13A. For zooming in and zooming out of the visual field on the visual field image 46, a possible way is to advance and retract the endoscope 24 with respect to the eyeball 30.

Thus, the drive controller 68 of the controller 28 controls the movement of the endoscope 24 through the arm part 42 so that the visual field of the endoscope 24 zooms in and out in accordance with the back and forth operation of the second joystick 54. For example, as illustrated in FIG. 2, the position of the endoscope 24 can be advanced and retracted (Zin, Zout) to cause the visual field image 46 to zoom in and out.

In addition, an image processor 70 included in the controller 28 performs image processing so that the visual field image 46 turns in accordance with left and right operations of the second joystick 54. The visual field image resulting from image processing is displayed on the monitor 38 via an image generator 72.

Thus, operation of the second joystick 54 is only needed to cause the visual field to zoom in and zoom out without regard to the movement of the endoscope 24, and the visual field image 46 can be turned by image processing without moving the endoscope 24. In other words, because the visual field image 46 can be changed in accordance with the operation of the second joystick 54 on the foot controller 26, interruption of manual work of the doctor 18, who is the operator, to change the visual field image 46 can be avoided.

### [Operation in emergency]

When the kick switch 66 of the foot controller 26 is operated in an emergency, the surgery assistance device 14 according to the embodiment is capable of retracting the endoscope 24 from the eyeball 30 by the retracting mechanism 110 described above. In other words, in an emergency, the doctor 18 only needs to move his/her foot 56 horizontally to retract the endoscope 24 from the eyeball 30.

While the present invention has been described above with reference to the embodiment, the present invention is not limited to the embodiment, and any combination or substitution of components in the embodiment as appropriate is included in the present invention. In addition, modifications such as combinations, changes in the order of processes, and various changes in design in the embodiment may be made on the embodiment on the basis of knowledge of a person skilled in the art, and such modified embodiments may be within the scope of the present invention.

### Industrial Applicability

The present invention can be used for a foot controller used to operate and control medical surgical tools during surgery.

### Reference Signs List

- 10: surgical system
- 12: operating table
- 14: surgery assistance device
- 16: patient
- 18: doctor
- 20: treatment tool
- 22: endoscope holder
- 24: endoscope
- 26: foot controller
- 28: controller
- 30: eyeball
- 38: monitor
- 40: base part
- 42: arm part
- 44: holding member
- 46: endoscopic image
- 48: housing
- 50: placement part
- 52: first joystick
- 54: second joystick
- 56: foot
- 62: recess
- 66: kick switch
- 68: drive controller
- 70: image processor
- 110: retracting mechanism

## Claims

1. A surgery assistance device comprising:
an endoscope (24);
a holding member (22) for holding the endoscope (24);
an arm part (42) for adjusting a position of the holding member (22);
a display (38) configured to display a visual field image taken by the endoscope (24);
a foot controller (26); and
a controller (28) configured to change the visual field image in accordance with an operation on the foot controller (26),
wherein the foot controller (26) comprises:
a placement part (50) on which a foot is to be placed;
a first operating part (52) capable of being operated back and forth and left and right by the foot placed at a first position (P1) of the placement part (50); and
a second operating part (54) capable of being operated back and forth and left and right by the foot placed at a second position (P2) of the placement part (50), wherein
on the placement part (50), the first operating part (52) and the second operating part (54) are arranged along a front-back direction, and
a predetermined operation of the device is selected in accordance with a direction of an operation on the first operating part (52) or the second operating part (54),
wherein the controller (28) includes:
a drive controller (68) configured to control movement of the endoscope (24) through the arm part (42) so that the visual field of the endoscope (24) zooms in and out in accordance with the back and forth operation of the second operating part (54); and
an image processor (70) configured to perform image processing so that the visual field image turns in accordance with a left/right operation of the second operating part (54).

2. The surgery assistance device according to claim 1, wherein the drive controller (68) is configured to control movement of the endoscope (24) through the arm part (42) so that a visual field of the endoscope (24) moves in accordance with a back/forth/left/right operation of the first operating part (52).

3. The surgery assistance device according to any one of claims 1 or 2, wherein
the first operating part (52) is located at a recess (62) on the placement part (50), the recess (62) being at a position lower than the first position, and
the second operating part (54) is located at a position that does not interfere with the foot placed at the first position.

4. The surgery assistance device according to any one of claims 1 to 3, wherein
the second operating part (54) is located at a higher position than the first operating part (52), and
the first operating part (52) is located at a position that does not interfere with the foot placed at the second position.

5. The surgery assistance device according to any one of claims 1 to 4, further comprising:
a third operating part (66) on a side of the placement part (50), wherein
the third operating part (66) is capable of being operated by a horizontal movement of the foot placed at the first position and also capable of being operated by a horizontal movement of the foot placed at the second position.

6. The surgery assistance device according to claim 5, wherein
the third operating part (66) is a pair of switches at respective sides of the placement, and
the first operating part (52) and the second operating part (54) are located in a region between the pair of switches.

7. The surgery assistance device according to any one of claims 1 to 6, wherein
the first operating part (52) and the second operating part (54) are joysticks.

## Patentansprüche

1. Chirurgie-Assistenzvorrichtung, enthaltend:
ein Endoskop (24);
ein Halteelement (22) zum Halten des Endoskops (24);
ein Armteil (42) zum Einstellen einer Position des Halteelements (22);
eine Anzeige (38), die konfiguriert ist, ein von dem Endoskop (24) aufgenommenes Sichtfeldbild anzuzeigen;
eine Fußsteuerung (26); und
eine Steuerung (28), die konfiguriert ist, das Sichtfeldbild gemäß einer Betätigung an der Fußsteuerung (26) zu verändern,
wobei die Fußsteuerung (26) enthält:
ein Platzierungsteil (50), auf das ein Fuß zu platzieren ist;
ein erstes Betätigungsteil (52), das in der Lage ist, hin und her sowie links und rechts mit dem Fuß betätigt zu werden, der in einer ersten Position (P1) des Platzierungsteils (50) platziert ist; und
ein zweites Betätigungsteil (54), das in der Lage ist, hin und her sowie links und rechts mit dem Fuß betätigt zu werden, der in einer zweiten Position (P2) des Platzierungsteils (50) platziert ist, wobei
an dem Platzierungsteil (50) das erste Betätigungsteil (52) und das zweite Betätigungsteil (54) entlang einer Vorne-Hinten-Richtung angeordnet sind, und
eine vorbestimmte Betätigung der Vorrichtung gemäß einer Richtung einer Betätigung an dem ersten Betätigungsteil (52) oder dem zweiten Betätigungsteil (54) ausgewählt wird,
wobei die Steuerung (28) enthält:
eine Antriebssteuerung (68), die konfiguriert ist, eine Bewegung des Endoskops (24) durch das Armteil (42) zu steuern, so dass das Sichtfeld des Endoskops (24) gemäß der Hin- und Her-Betätigung des zweiten Betätigungsteils (54) hinein- und herauszoomt; und
einen Bildprozessor (70), der konfiguriert ist, eine Bildverarbeitung durchzuführen, so dass sich das Sichtfeldbild gemäß einer Links-/Rechts-Betätigung des zweiten Betätigungsteils (54) dreht.

2. Chirurgie-Assistenzvorrichtung nach Anspruch 1, wobei
die Antriebssteuerung (68) konfiguriert ist, die Bewegung des Endoskops (24) durch das Armteil (42) zu steuern, so dass sich ein Sichtfeld des Endoskops (24) gemäß einer Vor-/Rück-/Links-/Rechtsbetätigung des ersten Betätigungsteils (52) bewegt.

3. Chirurgie-Assistenzvorrichtung nach einem der Ansprüche 1 oder 2, wobei
das erste Betätigungsteil (52) in einer Ausnehmung (62) am Platzierungsteil (50) positioniert ist, wobei die Ausnehmung (62) sich in einer Position befindet, die niedriger ist als die erste Position, und
das zweite Betätigungsteil (54) in einer Position positioniert ist, die den in der ersten Position platzierten Fuß nicht beeinträchtigt.

4. Chirurgie-Assistenzvorrichtung nach einem der Ansprüche 1 bis 3, wobei
das zweite Betätigungsteil (54) in einer höheren Position als das erste Betätigungsteil (52) positioniert ist, und
das erste Betätigungsteil (52) in einer Position positioniert ist, die den Fuß in der zweiten Position nicht beeinträchtigt.

5. Chirurgie-Assistenzvorrichtung nach einem der Ansprüche 1 bis 4, ferner enthaltend:
ein drittes Betätigungsteil (66) an einer Seite des Platzierungsteils (50), wobei
das dritte Betätigungsteil (66) fähig ist, durch eine horizontale Bewegung des in der ersten Position platzierten Fußes betätigt zu werden und auch fähig ist durch eine horizontale Bewegung des in der zweiten Position platzierten Fußes betätigt zu werden.

6. Chirurgie-Assistenzvorrichtung nach Anspruch 5, wobei
das dritte Betätigungsteil (66) ein Paar Schalter an den jeweiligen Seiten der Platzierung ist, und
das erste Betätigungsteil (52) und das zweite Betätigungsteil (54) in einem Bereich zwischen dem Paar Schalter positioniert sind.

7. Chirurgie-Assistenzvorrichtung nach einem der Ansprüche 1 bis 6, wobei
das erste Betätigungsteil (52) und das zweite Betätigungsteil (54) Joysticks sind.

## Revendications

1. Un dispositif d'assistance chirurgicale comprenant :
un endoscope (24) ;
un élément de maintien (22) pour tenir l'endoscope (24) ;
une partie de bras (42) pour régler une position de l'élément de maintien (22) ;
un écran (38) configuré pour afficher une image de champ visuel prise par l'endoscope (24) ;
une commande au pied (26) ; et
une commande (28) configurée pour modifier l'image du champ visuel conformément à une opération sur la commande au pied (26),
dans lequel la commande au pied (26) comprend :
une partie de placement (50) sur laquelle un pied doit être placé ;
une première partie de manœuvre (52) capable d'être manœuvrée d'avant en arrière et de gauche à droite par le pied placé à une première position (P1) de la partie de placement (50) ; et
une seconde partie de manœuvre (54) capable d'être manœuvrée d'avant en arrière et de gauche à droite par le pied placé dans une seconde position (P2) de la partie de placement (50), dans lequel
sur la partie de placement (50), la première partie de manœuvre (52) et la seconde partie de manœuvre (54) sont disposées dans une direction avant-arrière, et
un manœuvre prédéterminé du dispositif est sélectionné conformément à une direction de manœuvre sur la première partie de manœuvre (52) ou la seconde partie de manœuvre (54),
dans lequel la commande (28) comprend :
une commande d'entraînement (68) configurée pour commander le mouvement de l'endoscope (24) à travers la partie du bras (42) de sorte que le champ visuel de l'endoscope (24) effectue un zoom avant et arrière conformément au va-et-vient manœuvre de la seconde partie de manœuvre (54) ; et
un processeur d'image (70) configuré pour effectuer un traitement d'image de sorte que l'image du champ visuel tourne conformément à un de manœuvre gauche/droite de la seconde partie de manœuvre (54).

2. Un dispositif d'assistance chirurgicale selon la revendication 1, dans lequel
la commande d'entraînement (68) est configurée pour commander le mouvement de l'endoscope (24) à travers la partie du bras (42) de sorte qu'un champ visuel de l'endoscope (24) se déplace conformément à un manœuvre arrière/avant/gauche/droite de la première partie de manœuvre (52).

3. Un dispositif d'assistance chirurgicale selon l'une quelconque des revendications 1 ou 2, dans lequel
la première partie de manœuvre (52) est située à un évidement (62) sur la partie de placement (50), l'évidement (62) étant à une position inférieure à la première position, et
la seconde partie de manœuvre (54) est située dans une position qui n'interfère pas avec le pied placé dans la première position.

4. Un dispositif d'assistance chirurgicale selon l'une quelconque des revendications 1 à 3, dans lequel
la seconde partie de manœuvre (54) est située à une position plus élevée que la première partie de manœuvre (52), et
la première pièce de manœuvre (52) est située dans une position qui n'interfère pas avec le pied placé dans la seconde position.

5. Un dispositif d'assistance chirurgicale selon l'une quelconque des revendications 1 à 4, comprenant en outre :
une troisième partie de manœuvre (66) sur un côté de la partie de placement (50), dans lequel
la troisième partie de manœuvre (66) est capable d'être manœuvrée par un mouvement horizontal du pied placé en première position et est capable d'être manœuvrée également par un mouvement horizontal du pied placé en seconde position.

6. Le dispositif d'assistance chirurgicale selon la revendication 5, dans lequel
la troisième partie de manœuvre (66) est une paire d'interrupteurs situés sur les côtés respectifs du placement, et
la première partie de manœuvre (52) et la seconde partie de manœuvre (54) sont situées dans une région située entre la paire d'interrupteurs.

7. Le dispositif d'assistance chirurgicale selon l'une quelconque des revendications 1 à 6, dans lequel
la première partie de manœuvre (52) et la seconde partie de manœuvre (54) sont des joysticks.
